# EUROPÄISCHE PATENTSCHRIFT

(11) **EP 0 398 460 B1**
(45) Veröffentlichungstag und Bekanntmachung des Hinweises auf die Patenterteilung: **02.07.1997**
(21) Anmeldenummer: 90250127.9
(22) Anmeldetag: 16.05.1990
(51) Int. Cl.: A61K 31/585

(54) **Dihydrospirorenon als Antiandrogen**
Use of dihydrospirorenone as an anti-androgen
Utilisation de la dihydrospirorénone comme agent antiandrogène

(30) Priorität: 16.05.1989 DE 3916112
(43) Veröffentlichungstag der Anmeldung: 22.11.1990
(73) Patentinhaber: SCHERING AKTIENGESELLSCHAFT, 13342 Berlin (DE)
(72) Erfinder: Beier, Sybille, Dr., D-1000 Berlin 31 (DE); Elger, Walter, Dr., D-1000 Berlin 33 (DE); Nishino, Yukishige, Dr., D-1000 Berlin 22 (DE); Wiechert, Rudolf, Prof.-Dr.-Dr., D-1000 Berlin 39 (DE)

(56) Entgegenhaltungen:
- EP-A- 0 253 607
- DE-A- 3 022 337
- US-A- 4 347 245
- KLINISCHE WOCHERSCHRIFT, Band 64, Nr. 16, 1986, Seiten 732-737; M. BREINER et al.: "Inhibition of androgen receptor binding by natural and synthetic steroids in cultured human genital skin fibroblasts"

## Beschreibung

Die vorliegende Erfindung betrifft die Verwendung der Verbindung der Formel I 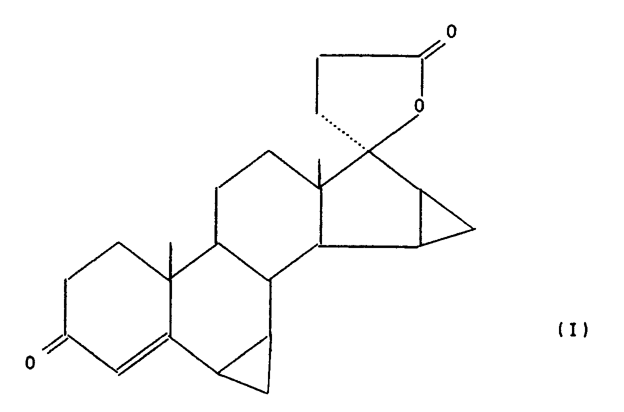 zur Herstellung eines Arzneimittels.

Die Verbindung I (Dihydrospirorenon) ist in der DE-A 26 52 761 unter anderen als Diuretikum vom Typ der Aldosteron-Antagonisten beschrieben.

Aus der DE-A 30 22 337 geht hervor, das die Verbindung I bei Dosierungen, bei denen die Antialdosteronwirkung bereits in Erscheinung tritt, auch eine deutliche gestagene Wirkung aufweist. Die Verbindung I kann daher alleine oder in Kombination mit Estrogenen in Präparaten zur Kontrazeption verwendet werden.

Gemäß der DE-A 30 22 337 sollen diese Präparate bei Frauen eingesetzt werden, die eine Kontrazeption wünschen und unter Bluthochdruck leiden oder bei denen es unter Einnahme oraler Kontrazeptiva zu Blutdruckanstiegen kommt. Damit wurde auch bei bezüglich eines erhöhten Blutdrucks prädisponierten Frauen eine hormonelle Kontrazeption möglich.

Ein Kombinationspräparat zur Substitutionstherapie und Kontrazeption für Frauen vor der Menopause (ab etwa dem 40. Lebensjahr) ist aus der EP-A 0253 607 bekannt. Dieses Kombinationspräparat enthält ein Estrogen aus der Gruppe
17β-Estradiol,
Ethinylestradiol und
Mestranol
sowie ein Gestagen aus der Gruppe
Levonorgestrel,
Gestoden,
Desogestrel,
3-Ketodesogestrel und
Norethindron.
Eine so gewählte Zusammensetzung soll hormonelle Unregelmäßigkeiten in der Übergangsphase der Premenopause ausgleichen und die durch die hormonelle Umstellung des weiblichen Organismus in dieser Phase bedingten Beschwerden lindern helfen. Gleichzeitig gewährleistet eine derartige Zusammensetzung den in diesem Lebensalter noch nötigen kontrazeptiven Schutz.

Aus verschiedenen, bekannten Gründen und der Zunahme der Inzidenz von Kontraindikationen mit steigendem Alter wird die Einnahme hormoneller Kontrazeptiva nur für Frauen etwa zum 35. Lebensjahr empfohlen, so daß eine hormonelle Behandlung in der Premenopause und eine Substitutionstherapie im Klimakterium unter Anwendung von Dosierungen, die gleichzeitig empfängnisverhütend wirken als problematisch angesehen werden kann.

Neben diesen eine Kontraindikation rechtfertigenden Umständen werden bei Frauen im fortgeschrittenen Alter häufig Androgenisierungserscheinungen wie beispielsweise Bartwuchs, Vertiefung der Stimme sowie auch unreine Haut beobachtet; außerdem ist oft ein Blutdruckanstieg zu verzeichnen.

Es wurde nun gefunden, daß die Verbindung der Formel I zusätzlich zu ihrer Gestagen- und Antialdosteronwirkung überraschenderweise eine starke anti-androgene Wirkungskomponente aufweist, und zwar ebenfalls bei Dosierungen, die die Formulierung dieser Verbindung als orale Kontrazeptiva zulassen. Dihydrospirorenon ist etwa gleichstark antiandrogen wirksam wie das als Standardverbindung geltende Cyproteronacetat (gleiche Maximalwirkung). (Tiermodell: juvenile, kastrierte und testosteron substituierte männliche Ratte; Methoden beschrieben in: Methods in Hormone Research, Editor: R.I. Dorfman, Academic Press, New York, London, 1969, pp. 241; or Androgens and Antiandrogens, Editors: L. Martin and M. Motta, Raven Press, New York, 1977, pp. 163).
Gegenstand der vorliegenden Erfindung ist somit die Verwendung der Verbindung der Formel I zur Herstellung eines Arzneimittels, welches zur gleichzeitigen Behandlung hormoneller Unregelmäßigkeiten in der Premenopause (Zyklusstabilisierung) und zur hormonellen Substitutionstherapie im Klimakterium und zur Behandlung androgeninduzierter Störungen und zur Kontrazeption geeignet ist.

Selbstverständlich kann ein erfindungsgemäß hergestelltes Arzneimittel auch ausschließlich zur Behandlung androgeninduzierter Störungen verwendet werden oder zur Behandlung solcher Störungen unter gleichzeitiger Kontrazeption und/oder Behandlung hormoneller Unregelmäßigkeiten in der Premenopause oder zur Behandlung solcher Störungen unter gleichzeitiger Kontrazeption und/oder zur hormonellen Substitutionstherapie im Klimakterium oder zur Behandlung solcher Störungen und/oder Behandlung hormoneller Unregelmäßigkeiten in der Premenopause und/oder zur hormonellen Substitutionstherapie im Klimakterium dienen.

Vorzugsweise wird gemeinsam mit einer Verbindung der Formel I ein Estrogen verwendet. Ob eher ein synthetisches oder eher ein natürliches Estrogen verwendet wird, hängt davon ab, ob die kontrazeptive Wirkung oder der substitive Effekt im Vordergrund steht: im ersten Fall ist Ethinylestradiol oder ein anderes synthetisches Estrogen bevorzugt, im zweiten Fall sollte ein derartiges Arzneimittel ein natürliches Estrogen enthalten.

In jedem Fall aber gewährleistet ein derartiges Arzneimittel einer Frau im mittleren Lebensalter (ca. 35. - 55. Lebensjahr) eine Stabilisierung ihres Menstruationszyklus sowie die in diesem Lebensalter noch unabdingbare Kontrazeption unter gleichzeitiger günstiger Beeinflussung androgen-induzierter Störungen. Selbstverständlich ist dieses Arzneimittel auch für jüngere Frauen geeignet, insbesondere für solche, die eine spezielle Prädisposition für Bluthochdruck haben und unter Androgenisierungserscheinungen leiden.
Dabei wird eine derartige Verwendung erst möglich, weil die Verbindung der Formel I gleichzeitig eine gestagene, antialdosterone sowie starke antiandrogene Wirkung in sich vereinigt. Es war bisher kein Stoff bekannt, der diese drei Eigenschaften gleichzeitig aufweist.
Die Dosierung der Verbindung der Formel I soll 0,5 - 50 mg pro Tag, vorzugsweise 1 - 10 mg pro Tag, betragen.

Als Estrogene kommen alle bisher gebräuchlichen Estrogene in Betracht. Dabei sollte das angewandte Estrogen vorzugsweise in solchen Dosen verabreicht werden, daß die erfindungsgemäß eingesetzte Estrogenmenge gleich der ist, die der Verabfolgung von 0,02 bis 0,04 mg 17α-Ethinylestradiol bzw. 0,5 bis 4,0 mg Estradiolvalerat täglich entspricht. Als Estrogenkomponente sind unter anderem auch die 17α-Ethinylestradiol-ester und -äther geeignet sowie zum Beispiel Ester des 17α-Ethinyl-7α-methyl-1,3,5(10)-estratrien-1,3,17β-triols (deutsche Patentschrift 1 593 509 und deutsche Offenlegungsschrift 2 818 164). Ferner auch noch die in der DE-A 36 28 189 beschriebenen 14,17β-Ethano-14β-estratriene. Die estrogenen und gestagenen Wirkstoffkomponenten werden vorzugsweise zusammen oral appliziert; sie können jedoch auch getrennt und/oder parenteral bzw. transdermal verabreicht werden.

Die Formulierung der erfindungsgemäßen Präparate auf Basis von 6β,7β;15β,16β-Dimethylen-3-oxo-4-androsten-[17(β-1′]-perhydrofuran-2′-on erfolgt in an sich bekannter Weise, indem der Wirkstoff, gegebenenfalls in Kombination mit einem Estrogen, mit den in der Galenik gebräuchlichen Trägersubstanzen, Verdünnungsmitteln, gegegebenenfalls Geschmackskorrigentien usw., verarbeitet und in die gewünschte Applikationsform überführt wird. Für die bevorzugte orale Applikation kommen insbesondere Tabletten, Dragees, Kapseln, Pillen, Suspensionen oder Lösungen infrage. Für die parenterale Applikation sind insbesondere ölige Lösungen, wie zum Beispiel Lösungen in Sesamöl, Rizinusöl und Baumwollsamenmöl geeignet. Zur Erhöhung der Löslichkeit können Lösungsvermittler, wie zum Beispiel Benzylbenzoat oder Benzylalkohol, zugesetzt werden.

Nachfolgend wird die Formulierung einiger Präparate anhand verschiedener Ausführungsbeispiele näher erläutert:

### Beispiel 1

20,0 mg 6β,7β;15β,16β-Dimethylen-3-oxo-4-androsten-[17(β-1′)-spiro-5′]-perhydrofuran-2′-on und 0,05 mg 17α-Ethinylestradiol werden mit 140,45 mg Milchzucker, 59,5 mg Maisstärke, 2,0 mg Aerosil, 2,5 mg Polyvinylpyrrolidon 25 und 0,5 mg Magnesiumstearat homogen gemischt und ohne vorherige Granulierung zu einer Tablette von 225 mg Endgewicht gepreßt.

### Beispiel 2

Analog Beispiel 1 werden 10 mg 6β,7β;15β,16β-Dimethylen-3-oxo-4-androsten-[17(β-1′)-spiro-5′]-perhydrofuran-2′-on und 0,05mg 17α-Ethinylestradiol mit 150,45 mg 17α-Ethinylestradiol mit 150,45 mg Milchzucker, 59,5 mg Maisstärke, 2,0 mg Aerosil, 2,5 mg Polyvinylpyrrolidon 25 und 0,5 mg Magnesiumstearat zu Tabletten mit einem Endgewicht von 225 mg gepreßt.

### Beispiel 3

Analog Beispiel 1 werden 20 mg 6β,7β;15β,16β-Dimethylen-3-oxo-4-androsten-[17(β-1′)-spiro-5′]-perhydrofuran-2′-on mit 140,5 mg Milchzucker, 59,5 mg Maisstärke, 2,0 mg Aerosil, 2,5 mg Polyvinylpyrrolidon 25 und 0,5 mg Magnesiumstearat zu Tabletten mit einem Endgewicht von 225 mg gepreßt.

## Patentansprüche

1. Verwendung von Dihydrospirorenon zur Herstellung eines Arzneimittels, welches zur gleichzeitigen Behandlung von
a) aldosteroninduzierten Störungen
und
b) hormonellen Unregelmäßigkeiten (Zyklusstabilisierung)
und
c) zur Kontrazeption
und
d) zur Behandlung androgeninduzierter Störungen
geeignet ist.

2. Verwendung von Dihydrospirorenon zur Herstellung eines Arzneimittels, welches zur gleichzeitigen Behandlung von
a) aldosteroninduzierten Störungen
und
b) androgeninduzierten Störungen
und
c) zur hormonellen Substitutionstherapie im Klimakterium
geeignet ist.

3. Verwendung nach einem der Ansprüche 1 oder 2, wobei die aldosteroninduzierte Störung
Bluthochdruck ist.

4. Verwendung von Dihydrospirorenon nach einem der Ansprüche 1 oder 2 in Kombination mit
einem Estrogen.

5. Verwendung nach Anspruch 4, in der das Estrogen ein synthetisches Estrogen ist.

6. Verwendung nach Anspruch 4, in der das Estrogen ein natürliches Estrogen ist.

7. Verwendung der Kombination nach einem der Ansprüche 1 oder 4 zur Herstellung eines Arzneimittels für die premenopausale Frau.

## Claims

1. Use of dihydrospirorenone in the preparation of a medicament that is suitable for the simultaneous treatment of
a) aldosterone-induced disorders, and
b) hormonal irregularities (stabilisation of the cycle), and
c) for contraception, and
d) for the treatment of androgen-induced disorders.

2. Use of dihydrospirorenone in the preparation of a medicament that is suitable for the simultaneous treatment of
a) aldosterone-induced disorders, and
b) androgen-induced disorders, and
c) for hormone replacement therapy in the menopause.

3. Use according to either claim 1 or claim 2, wherein the aldosterone-induced disorder is high blood pressure.

4. Use of dihydrospirorenone according to either claim 1 or claim 2 in combination with an oestrogen.

5. Use according to claim 4, wherein the oestrogen is a synthetic oestrogen.

6. Use according to claim 4, wherein the oestrogen is a natural oestrogen.

7. Use of the combination according to either claim 1 or claim 4 in the preparation of a medicament for pre-menopausal women.

## Revendications

1. Utilisation de la dihydrospirorénone pour préparer un médicament qui convient au traitement simultané
a) des troubles induits par l'aldostérone
et
b) des dérèglements hormonaux (stabilisation du cycle)
et
c) à la contraception
et
d) au traitement des troubles induits par les androgènes.

2. Utilisation de la dihydrospirorénone pour préparer un médicament qui convient au traitement simultané
a) des troubles induits par l'aldostérone
et
b) des troubles induits par les androgènes
et
c) à la thérapie hormonale de substitution dans la ménopause.

3. Utilisation selon une des revendications 1 ou 2, dans laquelle la perturbation induite par l'aldostérone est l'hypertension sanguine.

4. Utilisation de la dihydrospirorénone selon une revendications 1 ou 2 en combinaison avec un oestrogène.

5. Utilisation selon la revendication 4, dans laquelle l'oestrogène est un oestrogène synthétique.

6. Utilisation selon la revendication 4, dans laquelle l'oestrogène est un oestrogène naturel.

7. Utilisation de la combinaison selon une des revendications 1 ou 4 pour préparer un médicament pour la femme préménopausée.
